# EUROPEAN PATENT APPLICATION

(11) **EP 1 382 363 A1**
(43) Date of publication of application: **21.01.2004**
(21) Application number: 02388046.1
(22) Date of filing: 15.07.2002
(51) Int. Cl.: A61M 5/172

(54) **Closed loop system for controlling blood glucose levels**

(71) Applicant: NOVO NORDISK A/S, 2880 Bagsvaerd (DK)
(72) Inventor: Storgaard-Larsen, Torben, 3050 Humlebaek (DK); Bielecki, Mia, 2000 Frederiksberg (DK); Panduro, Rasmus, 1705 Copenhagen V (DK); Steenholt, Lars, 3050 Humlebaek (DK); Skyggebjerg, Ole, 2860 Soborg (DK); Ulrich, Finn, 3550 Slangerup (DK); Shalmi, Michael, 2900 Hellerup (DK); Dall, Mads Henrik, 2900 Hellerup (DK)

(57) **Abstract**

The present invention relates to closed loop drug delivery systems. More specifically, the invention relates to systems for controlling the infusion rate of a drug or other substance having a regulating effect, especially of blood glucose. Thus, in a first aspect the present invention provides closed loop system comprising a sensor assembly having a sensor system adapted for providing a sensor signal indicative of a glucose level in blood, flow means for establishing a flow of blood from a patient to the sensor assembly and for returning at least a portion of the blood back to the patient, control means adapted to receive the signals from the sensor system and generate command signals in response thereto in order to keep the blood glucose level of the patient within a desired range, as well as delivery means for delivering an amount of at least one drug having a blood glucose regulating effect, wherein operation of the delivery means is affected by the command signals.

## Description

### FIELD OF THE INVENTION

The present invention relates to closed loop drug delivery systems and corresponding methods. More specifically, the invention relates to systems and methods for controlling the infusion rate of a drug or other substance having a regulating effect, especially of blood glucose.

### BACKGROUND OF THE INVENTION

A specific type of polyneuropathy is observed to develop in patients that are treated within an intensive care unit (known as an ICU) for several days to weeks and this for a variety of primary injuries or illnesses. This polyneuropathy, known as "Critical Illness Polyneuropathy" (hereinafter also designated CIPNP) occurs in about 70% of patients who have the systemic inflammatory response syndrome (SIRS) (Zochodne DW et al. 1987 Polyneuropathy associated with critical illness: a complication of sepsis and multiple organ failure. Brain, 110: 819-842); (Leijten FSS & De Weerdt AW 1994 Critical illness polyneuropathy: a review of the literature, definition and pathophysiology. Clinical Neurology and Neurosurgery, 96: 10-19).

However, clinical signs are often absent and it remains an occult problem in many ICUs worldwide. Nonetheless, it is an important clinical entity as it is a frequent cause of difficulty to wean patients from mechanical ventilation and it leads to problems with rehabilitation after the acute illness has been treated and cured.

When CIPNP is severe enough, it causes limb weakness and reduced tendon reflexes. Sensory impairment follows but is difficult to test in ICU patients. Electro-physiological examination (EMG) is necessary to establish the diagnosis (Bolton CF. 1999 Acute Weakness. In: Oxford Textbook of Critical Care; Eds. Webb AR, Shapiro MJ, Singer M, Suter PM; Oxford Medical Publications, Oxford UK; pp. 490-495), which will reveal a primary axonal degeneration of first motor and then sensory fibres. Phrenic nerves are often involved. Acute and chronic denervation has been confirmed in muscle biopsies of this condition. If the underlying condition (sepsis or SIRS) can be successfully treated, recovery from and/or prevention of the CIPNP can be expected. This will occur in a matter of weeks in mild cases and in months in more severe cases. In other words, the presence of CIPNP can delay the weaning and rehabilitation for weeks or months.

The pathophysiology of this type of neuropathy remains unknown (Bolton CF 1996 Sepsis and the systemic inflammatory response syndrome: neuromuscular manifestations. Crit Care Med. 24: 1408-1416). It has been speculated to be directly related to sepsis and its mediators. Indeed, cytokines released in sepsis have histamine-like properties which may increase micro-vascular permeability. The resulting endoneural oedema could induce hypoxia, resulting in severe energy deficits and hereby primary axonal degeneration. Alternatively, it has been suggested that cytokines may have a direct cytotoxic effect on the neurons. Contributing factors to disturbed microcirculation are the use of neuromuscular blocking agents and steroids. Moreover, a role for aminoglucosides in inducing toxicity and CIPNP has been suggested. However, there is still no statistical proof for any of these mechanisms in being a true causal factor in the pathogenesis of CIPNP.

Although polyneuropathy of critical illness was first described in 1985 by three different investigators, one Canadian, one American, and one French, to date there is no effective treatment to prevent or stop Critical Illness Polyneuropathy.

To date the current standard of practice of care, especially of critically ill patients, has been that within the settings of good clinical ICU practice, blood glucose levels are allowed to increase as high as to 250 mg/dL or there above. The reason for this permissive attitude is the thought that high levels of blood glucose are part of the adaptive stress responses, and thus do not require treatment unless extremely elevated (Mizock BA. Am J Med 1995; 98: 75-84). Also, relative hypoglycaemia during stress is thought to be potentially deleterious for the immune system and for healing (Mizock BA. Am J Med 1995; 98: 75-84).

Although the exact mechanisms behind CIPNP are thus not understood, a recent study (Van den Berghe G et al, Intensive insulin therapy in critically ill patients, the New England Journal of Medicine, Vol. 345, p. 1359-1367) suggests a specific therapy for critically ill patients.

More specifically, a prospective, randomized, controlled study was performed.

Methods: All mechanically ventilated, adult patients admitted to the intensive care unit (ICU) were eligible for inclusion. Only 5 patients participating in another trial and 9 who were moribund or DNR coded at ICU admission were excluded. At admission, patients were randomized to either strict normalization of glycaemia (4.5-6.1 mmol/L) with continuously infused insulin during intensive care, the 'intensive insulin schedule' (lls), or the currently used 'restrictive insulin schedule' (RIS), with insulin started when blood glucose exceeds 12 mmol/L in which case glycaemia is clamped to 10-12 mmol/L. An interim safety analysis revealed a difference in mortality, and the study was ended for ethical reasons.

Results: A total of 1548 patients were included, 765 in the IIS group, 783 in the RIS group, well matched at inclusion. IIS reduced ICU mortality by 43% (P=0.005) [63 deaths in the RIS group versus 35 in the IIS group; death odds ratio for IIS, corrected for all baseline univariate predictors of ICU death, was 0.52 (0.33-0.82), P=0.004] and hospital mortality by 34% (P=0.01). Mortality reduction occurred exclusively in long-stay ICU patients and was due to prevention of death from multiple organ failure with sepsis. IIS also reduced the incidence of blood stream infections, renal failure, anaemia and critical illness polyneuropathy as well as the need for dialysis or hemofiltration, red cell transfusion, prolonged mechanical ventilatory support and intensive care.

Conclusion: The data suggest that disturbances in glucose metabolism during critical illness are not "adaptive and beneficial" since strict metabolic control with exogenous insulin substantially reduces morbidity and mortality.

Further aspects of the above study as well as additional aspects in relation to CIPNP are described in WO 01/85256.

As appears from the above, strict metabolic control with exogenous insulin substantially reduces morbidity and mortality in critically ill patients. Consequently, it would be desirable if a corresponding treatment regimen could be offered to any critically ill patient which may benefice there from. However, in the above-referred study, insulin therapy was based on labour-intensive manual measurements of the glucose level and corresponding manual control of the insulin infusion rate.

Having regard to the above, it thus appears that the provision of an automated system providing both a glucose sensing means for monitoring the blood glucose level as well as means for operating an insulin pump in response hereto would be desirable, i.e. providing closed loop control of the blood glucose level of a patient. In this context closed-loop feedback refers to the automatic interaction between a sensor and an insulin pump. With a closed-loop system, a glucose sensor relays information to the pump, which will in turn deliver just the right amount of insulin. An open-loop system is one where the patient or the medical staff must decide the correct amount of insulin to deliver, based on the glucose information obtained, usually by a finger stick blood-glucose measurement.

In principle, such systems are known and have been proposed for the treatment of diabetes. For example US patent 4,245,634 discloses an artificial beta cell for regulating blood glucose concentration in a subject by continuously analyzing blood from the patient and deriving a computer output signal to drive a pump which infuses insulin at a rate corresponding to the signal. A value of blood glucose concentration from the analyzed blood is used by a computer to determine a rate of change of this concentration which in turn is used to derive a projected blood glucose level. This system was based on external blood glucose measurement and used relatively large amounts of blood.

A more recent system incorporating in principle the same components is marketed by Nikkiso (Japan) as an artificial endocrine pancreas Model STG-22 sold for emergency treatment for hyperglycaemia such as diabetic coma. This bedside-type artificial endocrine pancreas is a closed-loop glycaemic control system which achieves continuous blood glucose monitoring through the glucose sensor electrode via withdrawing of blood from a distal vein and automatically feed back insulin or glucose infusion to keep the appropriate blood glucose control. After the blood has been analyzed it is discarded.

The above-described systems may be considered as adapted primarily for hospital or clinical research use; however, it is not these kinds of systems which have been the objects of development during the last two decades. More specifically, most research and development has concentrated on the needs of the "typical" diabetes patient, i.e. it has been the object to provide a closed loop, automatic system which is as small and compact as possible allowing the system to be carried by the patient under the clothing or, ideally, implanted subcutaneously. Given these objects, *in vivo* glucose sensors have been proposed which are suitable for being fully implanted or arranged subcutaneously by means of a needle penetrating the skin. These sensors predominantly measure the glucose level in the interstitial fluid. Skin-mountable glucose sensors have also been proposed, however, these have not proven very reliable. The corresponding insulin pump to be used in combination with the glucose sensor may also be implanted or it may be carried externally by the patient in communication with the sensor.

A recently proposed closed loop system comprising a glucose sensor which is at least partially inserted into the body and in (cordless) communication with an external pump is disclosed in WO 00/74753.

The above considerations in respect of both the therapeutic approach proposed by Van den Berghe and the known systems adapted for glycaemic control in diabetic patients have focused on the hyperglycaemic "issue", i.e. they have addressed the concept of strict metabolic control using insulin to control hyperglycaemia. However, it may be argued that the results achieved in the Van den Berghe studies do not rely entirely (or primarily) on the glucose lowering effects of the treatment but do at least in part rely on "additional" beneficial effects achieved by infusing insulin. Although these effects are not well understood, it is assumed that insulin *per se* has a beneficial effect in the treatment of critically ill patients. Thus, following these thoughts, irrespective of whether the high levels of blood glucose often found in critically ill patients are the results of adaptive stress responses or are the results of relative insulin insufficiency, it was infusion of additional insulin *per se* which at least in part accounted for the results achieved.

### DISCLOSURE OF THE INVENTION

Having regard to the above, it is the object of the present invention to provide a system which is suitable for controlling blood glucose concentration in the body of a critically ill patient. The system should be simple and cost-effective to use and should impose a minimum of additional stress on the patient, this allowing the system to be applied in any given situation in which the attendant medical person may consider it to be of potential benefit for the patient.

More specifically, in a **first** aspect the present invention provides closed loop system comprising: a sensor assembly having a sensor system adapted for providing a sensor signal indicative of a glucose level in blood, flow means for establishing a flow of blood from a patient to the sensor assembly and for returning at least a portion of the blood back to the patient, control means adapted to receive the signals from the sensor system and generate command signals in response thereto in order to keep the blood glucose level of the patient within a desired range, as well as delivery means for delivering an amount of at least one drug having a blood glucose regulating effect, wherein operation of the delivery means is affected by the command signals.

Such a system provides a number of advantages over the prior art systems which makes it specifically suitable for the treatment of critically ill patients. By measuring the blood glucose level in blood, it is possible to achieve very accurate "real time" values, this in contrast to systems relying on interstitial fluid which will respond much slower to the often dramatic fluctuations which may occur in the blood glucose level of patients suffering from major metabolic disorders. A further problem with most *in vivo* placed glucose sensors is the need for re-calibration, as the conditions surrounding the sensor will change over time, often from hour to hour, this making re-calibration with freshly drawn blood necessary. Although it would be possible to arrange a sensor in the vasculature of the patient, also such sensors would need to be re-calibrated often just as they will have to be replaced at intervals which, indeed, would impose much more stress to the patient in contrast to the replacement of an external *in vitro* blood glucose sensor.

Although the present invention relies on continuous or quasi-continuous (or intermittent) withdrawal of blood from the patient, this is not considered an obstacle as a critically ill patient in a ICU almost always will have been provided with a permanent vascular access, for example in the form of a central venous catheter (CVC) which typically is provided with a plurality of lumens each being accessible via individual connectors. It should be noted that the term "quasi-continuous" is no precise definition but merely indicates that an action takes place at very short intervals, e.g. intermittently every 1 or 5 minute.

Although the above-described artificial endocrine pancreas from Nikkiso also relies on *in vitro* measuring of the blood glucose level, it is reported that for every single measurement 2 ml of blood will have to be withdrawn and subsequently discarded. Indeed, for continuous or quasi-continuous blood glucose monitoring this would, for example during a week of treatment, amount to a volume of discarded blood which would be entirely unacceptable for a critically ill patient.

The sensor assembly comprising the actual glucose sensor element(s) may have any suitable configuration. For example, it may comprise a flow-through sensor assembly which measures the glucose level "truly" continuously, i.e. as the blood is passed (slowly) past the sensor, or in a quasi-continuous fashion in which the flow of blood is stopped intermittently for a period of time necessary for the sensor to properly determine the glucose level. In a different arrangement the sensor assembly comprises additional means for drawing a (small) sample of blood into a chamber in which the sensor is arranged, this allowing the blood to flow continuously through the sensor assembly. The blood drawn into the chamber may be returned or it may be discarded after use. The latter would be relevant in case a sensor element in contact with the blood comprises chemical substances which may transfer to the blood during measuring.

In order to return the blood drawn from the patient back to the patient, the blood may be circulated in a loop or it may be withdrawn and returned back by reversing the flow direction, e.g. by using a purge fluid. Indeed, such a setup would only make possible intermittent or quasi-continuous blood glucose measuring.

In preferred embodiments, all blood contacting elements are provided as disposable structures, e.g. tubing, and sensor elements, preferably in the form of a single unit to be mounted in a single apparatus, just as the drug(s) having a blood glucose regulating effect should be provided in the form of prefilled replaceable cartridges or reservoirs. Such an apparatus may then comprise pump means cooperating with a portion of the tubing (or a pump integrated with the tubing) in order to establishing a flow of blood, sensor electronic means cooperating with the sensor element(s), delivery means for expelling the drug(s) from the car-tridge/reservoir, and control means for controlling the expelling rate in response to signals received from the sensor electronic means.

As stated above, the present invention addresses the problem of controlling blood glucose concentrations in the body of a critically ill patient, however, irrespective of the different embodiments and features described above, the closed loop system in accordance with the present invention can be used in accordance with different treatment regimens.

In accordance with a first treatment regimen, a drug having a blood glucose regulating effect is infused in order to control an undesired high level of blood glucose, the drug preferably comprising insulin. This regimen would correspond to the treatment proposed in the Van den Berghe studies.

In accordance with a second treatment regimen, insulin is infused, either in accordance with a preset rate or profile or by using a closed loop arrangement allowing an insulin "clamp" to be provided, in combination with glucose, wherein glucose is infused in order to control hypoglycaemia or provide a given desired blood glucose level above the "normal" level. For this second regimen the closed loop system in accordance with the invention comprises delivery means adapted for the controlled infusion of insulin as well as the glucose. The infusion of insulin may be performed manually, by automatic infusion means external to the closed loop system or formed integrally therewith.

In a broader aspect, the present invention provides closed loop system comprising: a sensor assembly having a sensor system adapted for providing a sensor signal indicative of a level of a blood parameter, flow means for establishing a flow of blood from a patient to the sensor assembly and for returning at least a portion of the blood back to the patient, control means adapted to receive the signals from the sensor system and generate command signals in response thereto in order to keep the level of the blood parameter of the patient within a desired range, as well as delivery means for delivering an amount of at least one drug having a regulating effect on the blood parameter, wherein operation of the delivery means is controlled by the command signals.

In a **second** aspect the present invention provides a method of treating a critically ill patient, CIPNP-patient or potential CIPNP-patient, comprising the steps of drawing blood from a patient, determining a value indicative of a glucose level in at least a portion of the withdrawn blood, returning at least a portion of the blood to the patient, infusing an effective amount of at least one drug having a blood glucose regulating effect into the patient in response to the determined value in order to keep the blood glucose level of the patient within a desired range.

In a broader aspect, the present invention provides a method of treating a critically ill patient, CIPNP-patient or potential CIPNP-patient, comprising the steps of drawing blood from a patient, determining a value indicative of level of a blood parameter in at least a portion of the withdrawn blood, returning at least a portion of the blood to the patient, infusing an effective amount of a drug having a regulating effect on the blood parameter into the patient in response to the determined value in order to keep the blood parameter level of the patient within a desired range.

In a further aspect the present invention provides a method of treating a critically ill patient, CIPNP-patient or potential CIPNP-patient, comprising the steps of infusing insulin, determining a value indicative of a glucose level in a body fluid of the patient, infusing an effective amount of at least one drug having a blood glucose increasing effect into the patient in response to the determined value in order to keep the blood glucose level of the patient within a desired range, the drug preferably being glucose.

As used herein, the term "drug having a blood glucose regulating effect" is meant to encompass any drug-containing flowable medicament having such an effect and capable of being passed through a delivery means such as a hollow needle or an infusion line in a controlled manner, such as a liquid, solution, gel or fine suspension. In the description of the preferred embodiments reference will be made to the use of insulin.

### BRIEF DESCRIPTION OF THE DRAWINGS

In the following the invention will be further described with references to the drawings, wherein
fig. 1 shows a schematic representation of a first embodiment of a closed loop system,
fig. 2 shows a schematic representation of a second embodiment of a closed loop system,
fig. 3 shows a schematic representation of a third embodiment of a closed loop system, and
fig. 4 shows a schematic representation of a fourth embodiment of a closed loop system.

### DESCRIPTION OF THE PREFERRED EMBODIMENTS

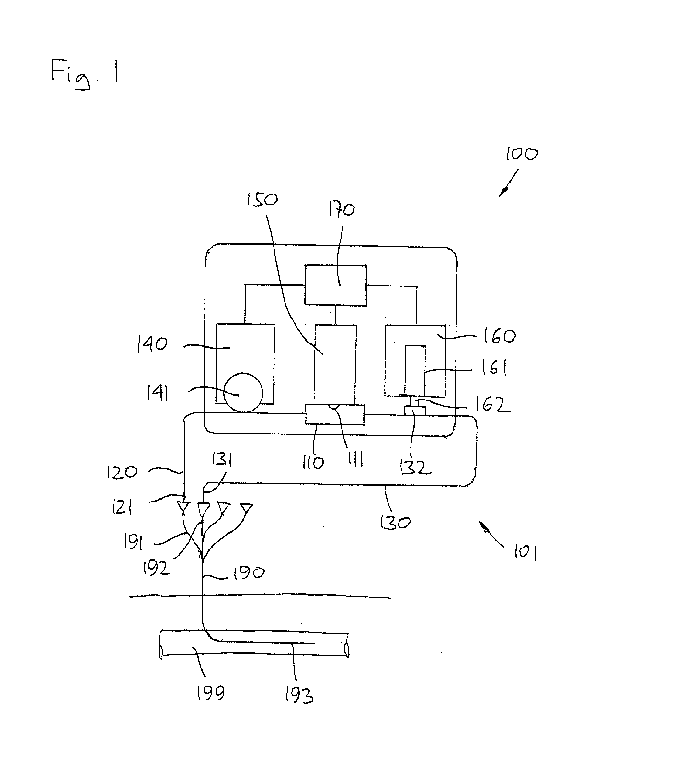

Fig. 1 shows a schematic representation of a **first** embodiment of a closed loop system for keeping the blood glucose level of a patient within a desired range in accordance with the invention, the system comprising a (disposable) fluid conduit assembly 101 and a control and actuation assembly 100 adapted to be in mating and cooperating engagement with each other in a situation of use.

More specifically, the fluid conduit assembly 101 comprises a sensor housing 110 having inlet and outlet openings and a flow path established there between and at glucose sensor element 111 disposed adjacent the fluid pathway, an inlet conduit 120 having a proximal inlet 121 and an outlet conduit 130 having a distal outlet 131, the conduits being connected to the inlet and outlet openings of the sensor housing, respectively to form a fluid conduit between the in- and outlets 121, 131. In this way a flow-through sensor unit is formed. The outlet conduit is provided with an inlet port 132 to allow a drug to be infused into the conduit there through. The fluid conduit assembly may be formed as an integral unit or the sensor housing and the inlet and outlet conduits may be provided as individual members adapted to be connected to each other.

The control and actuation assembly 100 comprises a pump assembly 140 for establishing a flow of blood from a patient to the sensor housing and for returning the blood to the patient. The pump is in the form of a peristaltic pump having a roller assembly 141 adapted to engage a portion of the inlet conduit 120, however, a peristaltic finger pump could also be used just as the inlet conduit may be provided with a membrane pump actuated by the pump assembly. The pump assembly may be operated to provide a constant flow rate or a variable flow rate in accordance with the requirements of the sensor system and/or the drug delivery means (to be described).

The control and actuation assembly 100 further comprises a sensor assembly 150 having sensor means adapted to cooperate with the sensor element 111 to form a sensor system for providing a sensor signal indicative of a glucose level in a fluid (e.g. blood) flowing through the sensor housing. The glucose sensor element typically comprises one or more reagents which react with the glucose in the fluid corresponding to the glucose concentration, this primary reaction being detected by the sensor means for providing a sensor signal indicative of the glucose level in a fluid. For example, glucose detection can be based upon depletion of oxygen during enzymatic oxidation of glucose, as described, e.g., in U.S. Patent 5,518,694. The sensor housing and the sensor assembly may be provided with additional sensors and corresponding sensor means for measuring further parameters of the blood, e.g. oxygen, carbon dioxide, pH, potassium, sodium, calcium etc.

Depending on the type of sensor(s) used, the glucose level may be measured "truly" continuously, i.e. as the blood is passed (slowly) past the sensor, or in a quasi-continuous fashion in which the pump assembly is operated to intermittently stop the flow of blood for a period of time necessary for the sensor to properly determine the glucose level.

Further, the control and actuation assembly 100 comprises a delivery assembly 160 for delivering an amount of at least one drug to a patient. More specifically, in the shown embodiment the delivery assembly 160 is adapted to releasably accommodate a drug reservoir 161 in the form of a conventional prefilled drug cartridge of the type used e.g. in combination with insulin delivery devices such as injection pens, i.e. a columnar reservoir comprising a displaceable piston. Correspondingly, the delivery assembly comprises a piston engaging member (not shown) adapted to engage and move forward the piston in order to expel the drug in a controlled way as will be explained in greater detail below.

In the shown embodiment, connecting means is provided allowing the drug cartridge to be connected in fluid communication with the inlet port 132 of the outlet conduit, this allowing the drug to be delivered directly to the blood without the need for additional tubing. Preferably, the inlet port comprises a self-sealing septum allowing the flow communication to be established using a double ended needle assembly 162 of the type used in combination with drug infusion systems of the pen type, this allowing for ease of operation.

In order to control operation of the above described subassemblies of the closed loop system in accordance with present invention, the control and actuation assembly 100 comprises a control assembly 170 adapted to receive signals from the sensor assembly and generate command signals in response thereto in order to keep the blood glucose level of the patient within a desired range, i.e. by controlling the delivery means and thereby the infusion rate of a drug having a blood glucose regulating effect, e.g. insulin.

In a preferred embodiment, the control assembly 170 is designed to model a pancreatic beta cell. In other words, the control assembly commands the delivery assembly to release insulin into the body of a patient at a rate (either continuously or intermittent (pulsed)) that causes the glucose concentration in the blood to be kept (or "clamped") within a desired range, e.g. 4.5-6.1 mmol/L. To provide a control algorithm to achieve this object is *per se* not an object of the present invention, however, such algorithms are generally well known as they have been the object of intensive research in the last two decades, see for example US patent 4,245,634 (disclosing the "Biostator"®, Ames Research) or more recently WO 00/74753 (Minimed Inc.). The control algorithm may rely solely on the sensed blood glucose or may incorporate additional measured values (e.g. by employing a sensor system comprising a plurality of different sensors) as well as non-measured values (i.e. manually input information in respect of e.g. carbohydrate intake) to allow for more sophisticated algorithms.

The control assembly may operate the pump assembly to provide either a constant or non-constant flow as required by the sensor assembly. In addition, the flow rate me be controlled as a function of the amount of drug infused.

Advantageously, the control assembly also controls a user interface (not shown) which may comprise display and input means allowing patient-specific settings to be entered or priming routines to be performed, or input/output means allowing the closed loop system to communicate with other systems for data up- or download. Further, the control assembly may be provided with means for controlling and checking the entire system and eventually to sound an alarm in case of a malfunction.

The in- and outlets 121, 131 of the fluid conduit assembly 101 are adapted for connection to catheter means configured for insertion into a blood vessel of the patient. In the shown embodiment, the in- and outlets 121, 131 are connected to respective proximal openings 191, 192 of a quad lumen catheter 190, e.g. as supplied by Arrow, the distal end 193 of the catheter being placed in the vasculature 199 of a patient.

In the shown embodiment the different subassemblies are incorporated into a single housing to form a single apparatus, however, if desired, the subassemblies could be in the form individual components to be connected by flow conduits and/or control cables. Correspondingly, additional subassemblies may be provided, either arranged within the common housing or provided as separate add-on units. For example, one or more further delivery assemblies may be provided for controlled delivery of one or more further drugs or substances, e.g. glucose allowing counter regulation of hypoglycaemia.

In use the system may be set up and operated in the following way. First, a fluid conduit assembly 101 is primed with a fluid such as saline. A drug cartridge of the cylinder/piston type is loaded into the delivery assembly and a double ended needle assembly 162 is mounted such that the first end of the needle penetrates a septum of the cartridge to establish fluid communication with the interior thereof. Thereafter the piston actuating means are advanced until a small amount of drug is expelled from the free second end of the needle (the so-called air-shot). Thereafter is the fluid conduit assembly mounted in and secured in place in the control and actuation assembly 100 such that the peristaltic pump means 141 engages the conduit 120, the sensor housing 110 engages the sensor means of the sensor assembly, and the free end of the needle is inserted into the drug inlet port 132. Finally, the in- and outlets 121, 131 of the fluid conduit assembly is connected to a catheter means in flow communication with the vasculature of the patient, e.g. a single multi-lumen catheter. In addition to the above procedures, it may be necessary to calibrate the sensor system.

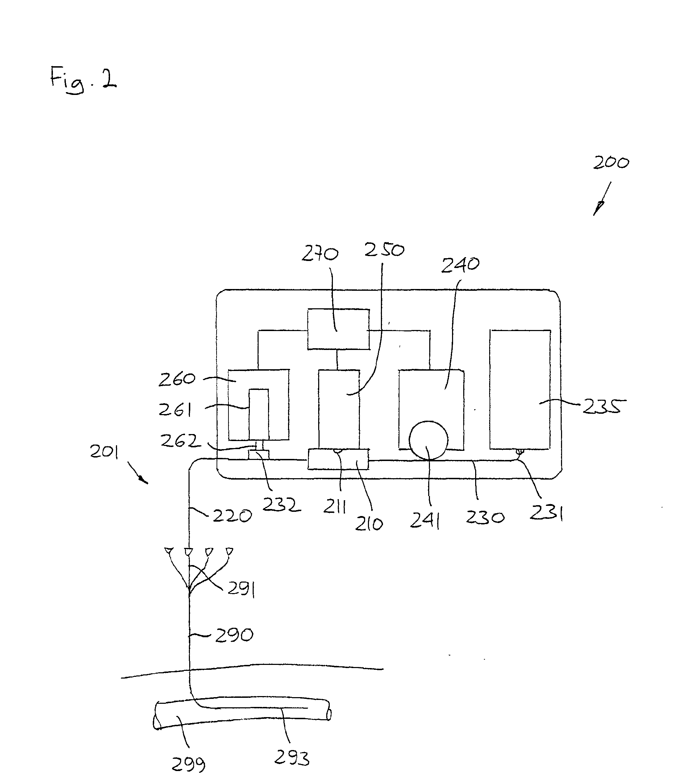

Fig. 2 shows a schematic representation of a second embodiment of a closed loop system for keeping the blood glucose level of a patient within a desired range in accordance with the invention, the system comprising a (disposable) fluid conduit assembly 201 and a control and actuation assembly 200 adapted to be in mating and cooperating engagement with each other in a situation of use.

More specifically, the fluid conduit assembly 201 comprises a sensor housing 210 having two openings and a flow path established there between and at glucose sensor element 211 disposed adjacent the fluid pathway, a first conduit 220 having an end 221 and a second conduit 230 having an end 231, the conduits being connected to the sensor housing to form a fluid conduit between the ends 221, 231. In this way a flow-through sensor unit is formed. The first conduit is provided with an inlet port 232 to allow a drug to be infused into the conduit there through. As described with reference to the first embodiment above, the fluid conduit assembly may be formed as an integral unit or the sensor housing and the first and second conduits may be provided as individual members adapted to be connected to each other.

In contrast to the first embodiment described above, an additional reservoir 235 comprising a purge fluid is provided in fluid communication with the end 231 of the second fluid conduit. The reservoir is preferably in the form of a conventional prefilled infusion liquid bag, the bag being suspended relative to the assembly 300 in any convenient way.

The control and actuation assembly 200 comprises a pump assembly 240 for establishing a flow of blood from a patient to the sensor housing and for returning the blood to the patient. Basically the pump assembly 240 corresponds to the pump assembly 140 described above and thus comprises a roller assembly 241 adapted to engage a portion of the first conduit 230, however, in contrast hereto the pump assembly is adapted for pumping fluid in either direction as will be explained in greater detail below.

The control and actuation assembly 200 further comprises a sensor assembly 250 having sensor means adapted to cooperate with the sensor element 211 to form a sensor system for providing a sensor signal indicative of a glucose level in a fluid (e.g. blood) accommodated in the sensor housing. Basically the sensor assembly 250 corresponds to the sensor assembly 250 described above, however, due to the way blood is transported to and from the sensor housing, the sensor system will most likely operate in a non-continuous way measuring the glucose level on non-flowing blood.

Further, the control and actuation assembly 200 comprises a delivery assembly 260 for delivering an amount of a drug to a patient. Basically the delivery assembly 260 corresponds to the delivery assembly 160 described above, i.e. comprising a cartridge 261 and a needle assembly 262 to cooperate with the inlet port 232, however, due to the way blood is transported to and from the sensor housing, the flow of drug only takes place when blood and purge fluid (to be described below) is pumped in a direction towards the patient.

In order to control operation of the above described subassemblies of the closed loop system in accordance with present invention, the control and actuation assembly 200 comprises a control assembly 270 adapted to receive signals from the sensor assembly and generate command signals in response thereto in order to keep the blood glucose level of the patient within a desired range, i.e. basically as described with reference to control assembly 170 above.

However, whereas the first embodiment of the closed loop system was adapted to circulate blood through the fluid conduit assembly 101, the second embodiment is operated to draw a sample of blood from the patient through the first conduit 220 and into the sensor housing 210, this allowing the blood to fill the sensor housing. To assure proper filling of the sensor housing and to prevent blood from entering the purge fluid reservoir, blood detection means (not shown) advantageously is provided in association with the second fluid conduit.

In order to return the blood drawn from the patient after glucose measurement has taken place, the pump direction is reversed whereby purge fluid is drawn from the reservoir 235 and pumped towards the patient. In this way the blood is returned to the patient and the sensor housing is purged with purge fluid (e.g. saline solution). Preferably, the delivery means 260 are operated just prior to reversing the flow, this allowing the drug to be infused during the purging action.

The end 221 serving as combined in- and outlet for the fluid conduit assembly 201 is adapted for connection to catheter means configured for insertion into a blood vessel of the patient. In the shown embodiment, the end 221 is connected to a proximal opening 291 of a quad lumen catheter 290, the distal end 293 of the catheter being placed in the vasculature 299 of a patient.

As for the first described embodiment, the different subassemblies as well as additional assemblies may be provided and connected to each other in any convenient way.

In use the system of the second embodiment may be set up and operated in the following way. First, a fluid conduit assembly 201 is connected to a reservoir 235 comprising the purge fluid by which the conduit is subsequently primed. A drug cartridge of the cylinder/piston type is loaded into the delivery assembly and a double ended needle assembly 262 is mounted such that the first end of the needle penetrates a septum of the cartridge to establish fluid communication with the interior thereof. Thereafter the piston actuating means are advanced until a small amount of drug is expelled from the free second end of the needle. Thereafter the fluid conduit assembly and purge fluid reservoir are mounted in and secured in place in the control and actuation assembly 200 such that the peristaltic pump means 241 engages the first conduit 230, the sensor housing 210 engages the sensor means of the sensor assembly 250, and the free end of the needle is inserted into the drug inlet port 232. Finally, the end 221 serving as combined in- and outlet for the fluid conduit assembly is connected to a catheter means in flow communication with the vasculature of the patient, e.g. a single multi-lumen catheter. In addition to the above procedures, it may be necessary to calibrate the sensor system.

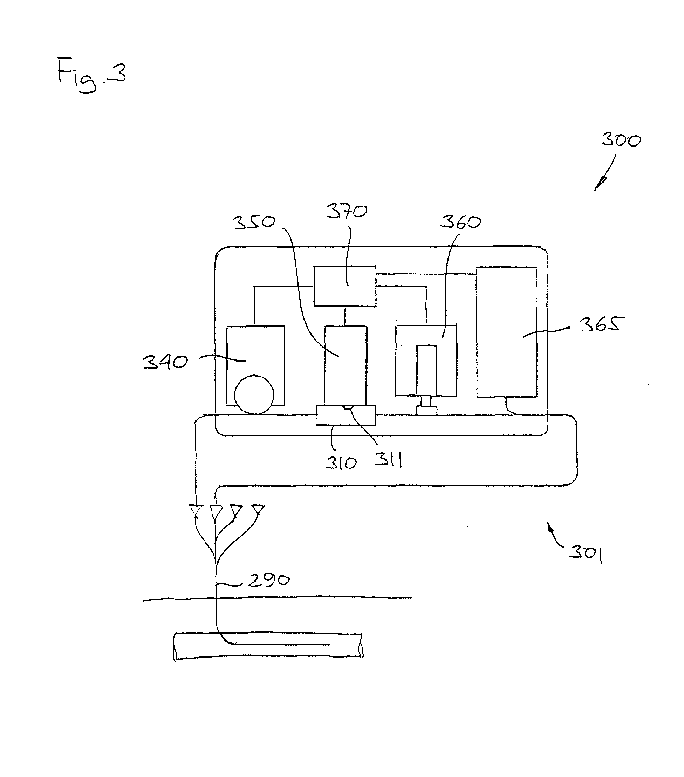

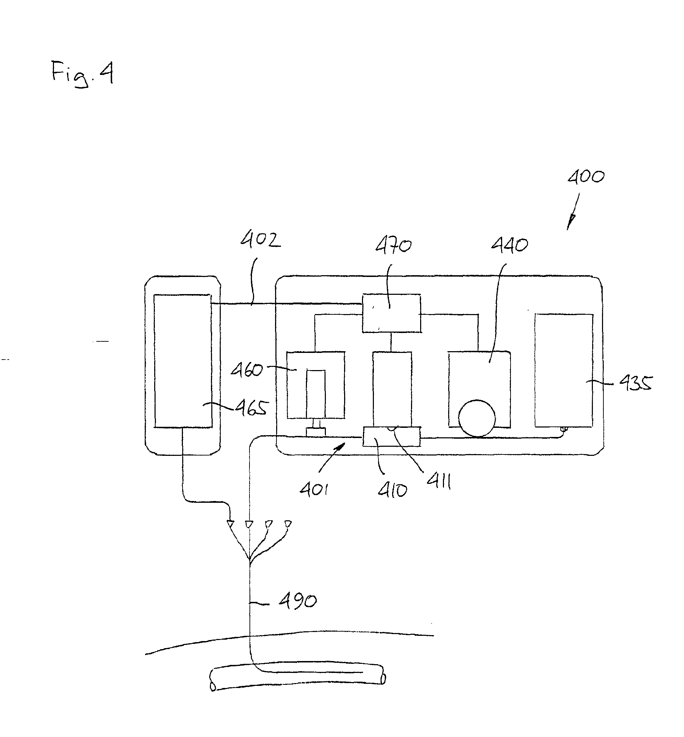

The systems described with reference to figs. 1 and 2 have been adapted in accordance with a first treatment regimen in which a drug (e.g. insulin) having a blood glucose regulating effect is infused in order to control an undesired high level of blood glucose. With reference to figs. 3 and 4 third and fourth embodiments of the systems will be described, these systems being adapted in accordance with a second treatment regimen in which insulin is infused in combination with glucose, and where glucose is infused in order to control hypoglycaemia or provide a given desired blood glucose level.

Fig. 3 shows a schematic representation of the **third** embodiment of a closed loop system. This embodiment is based on the first embodiment, however, the configuration and functionality of some of the components have been adapted to the intended situation of use just as additional components have been added. For the same or similar components corresponding reference numerals are used, i.e. 300 numbers instead of 100 numbers.

More specifically, the system comprises a control and actuation assembly 300 adapted to be in mating and cooperating engagement with a (disposable) fluid conduit assembly 301 comprising a sensor housing 310 with a sensor element 311. The control and actuation assembly 300 comprises a pump assembly 340 for establishing a flow of blood from a patient to the sensor housing and for returning the blood to the patient, a sensor assembly 350 having sensor means adapted to cooperate with the sensor element 311, and a first delivery assembly 360 for delivering insulin to a patient through multi lumen catheter 290. For a more detailed description of these components reference is made to the above description of the first embodiment.

In contrast to the first embodiment, the control and actuation assembly 300 comprises a second delivery assembly 365 for delivering a blood glucose elevating drug (e.g. glucose) to the patient. The second delivery assembly, which is only shown schematically, is arranged in fluid communication with the fluid conduit assembly and comprises a drug reservoir in association with expelling means adapted to expel the blood glucose elevating drug in a controlled way as will be explained in greater detail below.

The control and actuation assembly 300 further comprises a control assembly 370 adapted to receive signals from the sensor assembly and generate command signals in response thereto in order to keep the blood glucose level of the patient within a desired range, i.e. by controlling the second delivery means and thereby the infusion rate of the blood glucose elevating drug, typically glucose. Indeed, in this way the control means is only able to control and adjust a blood glucose level which otherwise would be below a desired range, e.g. 4.5-6.1 mmol/L.

As the control and actuation assembly 300 is adapted for the controlled infusion of insulin, the control assembly further comprises means for controlling the first delivery means and thereby the infusion rate of insulin. Insulin may be infused in accordance with a selected infusion rate (e.g. based on the body weight and the condition of the patient) or in accordance with a selected infusion profile (e.g. lower during the night). The insulin infusion rate may also be controlled by a control algorithm relying on a sensed blood insulin level (e.g. by providing the sensor system with means for detecting the blood insulin level) or may incorporate (additional) measured values (e.g. by employing a sensor system comprising a plurality of different sensors as well as body sensors for detecting e.g. EKG and body temperature) as well as non-measured values (i.e. manually input information in respect of e.g. carbohydrate intake) to allow for more sophisticated algorithms. As glucose is both infused and sensed, it follows that glucose should be infused downstream of the blood intake, this also applying to the fourth embodiment.

Fig. 5 shows a schematic representation of the fourth embodiment of a closed loop system. This embodiment is based on the second embodiment, however, the configuration and functionality of some of the components have been adapted to the intended situation of use just as additional components have been added. For the same or similar components corresponding reference numerals are used, i.e. 400 numbers instead of 200 numbers.

More specifically, the system comprises a control and actuation assembly 400 adapted to be in mating and cooperating engagement with a (disposable) fluid conduit assembly 401 comprising a sensor housing 410 with a sensor element 411. The control and actuation assembly 400 comprises a pump assembly 440 for establishing a flow of blood to and from a patient to the sensor housing and for returning the blood to the patient, a sensor assembly 450 having sensor means adapted to cooperate with the sensor element 411, and a first delivery assembly 460 for delivering insulin to a patient through multi lumen catheter 490, and a reservoir 435 comprising a purge fluid. For a more detailed description of these components reference is made to the above description of the first embodiment.

In contrast to the second embodiment, the control and actuation assembly 400 comprises a second delivery assembly 465 for delivering a blood glucose elevating drug (e.g. glucose) to the patient. The second delivery assembly, which is only shown schematically, is arranged in fluid communication with a multi lumen catheter 490 and comprises a drug reservoir in association with expelling means adapted to expel the blood glucose elevating drug in a controlled way as will be explained in greater detail below. In the shown embodiment the second delivery device is provided as an additional unit which is connected to the "main" assembly 400, power and control signals being provided from the latter through connecting means 402.

The control and actuation assembly 400 further comprises a control assembly 470 adapted to receive signals from the sensor assembly and generate command signals in response thereto in order to keep the blood glucose level of the patient within a desired range. This control assembly substantially controls infusion of the blood glucose elevating drug and insulin in the same way as for the third embodiment. As the blood glucose elevating drug is infused through the multi lumen catheter 490 in parallel with the blood drawn to and from the fluid conduit assembly 401, the second delivery assembly can be operated independently of the blood withdrawal and infusion means.

In the above description of the preferred embodiments, the different structures providing mechanical and electrical contact and communication between the different components just as the means providing the described functionality for the different components (i.e. dose setting, reservoir, energy source, memory, control means, display etc.) have been described to a degree to which the concept of the present invention will be apparent to the skilled reader.

The detailed construction and specification for the different structures are considered the object of a normal design procedure performed by the skilled person along the lines set out in the present specification.

## Claims

1. A closed loop system (100, 101) for controlling blood glucose concentration in the body of a patient, comprising:
- a sensor assembly (120) having a sensor system adapted for providing a sensor signal indicative of a glucose level in blood, the sensor system comprising a sensor element (11),
- flow means (120, 130, 140) for establishing a flow of blood from a patient to the sensor assembly and adapted for returning at least a portion of the blood to the patient,
- control means (170) adapted to receive the signals from the sensor system and generate command signals in response thereto in order to keep the blood glucose level of the patient within a desired range,
- delivery means (160) for delivering an amount of at least one drug having a blood glucose regulating effect, wherein operation of the delivery means is affected by the command signals.

2. A closed loop system according to claim 1, wherein the sensor assembly comprises an inlet and an outlet, the flow means providing a flow of blood there through.

3. A closed loop system according to claim 2, wherein the sensor assembly comprises a fluid pathway in fluid communication with the inlet and outlet, the sensor element being disposed adjacent the fluid pathway.

4. A closed loop system according to claim 3, wherein the sensor assembly comprises a fluid cavity in fluid communication with the inlet, the sensor system being disposed adjacent the fluid cavity, means being provided for drawing fluid into the fluid cavity and for returning or discarding the fluid.

5. A closed loop system (201, 300) according to claim 1, wherein the sensor assembly comprises a combined inlet and outlet, the flow means (310) being operatable to provide a flow of blood through the combined inlet and outlet to the sensor assembly and for returning at least a portion of the blood there through.

6. A closed loop system according to claim 5, wherein the sensor assembly comprises a further opening connectable to a reservoir (235) of purge fluid, the flow means being operatable to intermittently draw blood from the patient and to return it by purging the sensor assembly by means of a flow of the purge fluid.

7. A closed loop system according to any of the previous claims, wherein the inlet and outlet or the combined inlet and outlet are adapted for connection to catheter means (50, 250) configured for insertion into a blood vessel (60, 260) of the patient.

8. A closed loop system according to any of the previous claims, wherein the control means and the delivery means are adapted to deliver an amount of an insulin containing drug in order to keep the blood glucose level of the patient within a desired range.

9. A closed loop system according to any of the previous claims, wherein the control means and the delivery means are adapted to deliver an amount of a glucose containing drug in order to keep the blood glucose level of the patient within a desired range.

10. A closed loop system according to any of the previous claims, wherein the delivery means comprises first means for delivering an amount of a drug having a blood glucose lowering effect and second means for delivering an amount of a substance having a blood glucose elevating effect, wherein operation of at least one of the first and second means is affected by the command signals.

11. A closed loop system according to any of claims 1-7, wherein the delivery means comprises first means for delivering an amount of a drug having a blood glucose lowering effect and second means for delivering an amount of a substance having a blood glucose elevating effect, wherein operation of at least the second means is affected by the command signals.

12. A closed loop system according to any of the previous claims, wherein the control means is adapted to regulate the blood glucose level of a patient within the range of 4.5-6.1 mmol/L.

13. A method of treating critically ill patients, CIPNP-patients or potential CIPNP-patients, comprising:
- drawing blood from a patient,
- determining a value indicative of a glucose level in at least a portion of the blood,
- returning at least a portion of the blood to the patient,
- infusing an effective amount of a drug having a blood glucose regulating effect into the patient in response to the determined value in order to keep the blood glucose level of the patient within a desired range, preferably within the range of 4.5-6.1 mmol/L.

14. A method as defined in claim 13, wherein blood is drawn from and returned to the patient continuously, a value indicative of the glucose level being determined continuously or quasi-continuously.

15. A method as defined in claim 13, wherein blood is drawn from and returned to the patient intermittently.
